# FASCICULE DE BREVET EUROPEEN

(11) **EP 3 013 758 B1**
(45) Date de publication et mention de la délivrance du brevet: **13.09.2017**
(21) Numéro de dépôt: 14759256.2
(22) Date de dépôt: 30.06.2014
(51) Int. Cl.: C02F 3/32, C02F 3/28, C05F 17/00, C02F 11/04, C12M 1/107, C02F 103/20

(54) **INSTALLATION DE TRAITEMENT ET DE VALORISATION D'EFFLUENTS D'ELEVAGE COMPORTANT UNE METHANISATION, DES CULTURES DE MICRO-ALGUES ET DE MACROPHYTES, ET UNE LOMBRICULTURE**
ANLAGE ZUR BEHANDLUNG UND RÜCKGEWINNUNG VON TIEREXKREMENTEN MIT METHANISATION, KULTIVIERUNG VON MIKROALGEN UND MAKROPHYTES SOWIE WURMKULTUR
FACILITY FOR TREATING AND RECYCLING ANIMAL WASTE COMPRISING METHANISATION, CULTIVATION OF MICROALGAE AND MACROPHYTES, AND VERMICULTURE

(30) Priorité: 28.06.2013 FR 1301538
(43) Date de publication de la demande: 04.05.2016
(73) Titulaire: Skyworld International Overseas Limited, Hong Kong (CN); Guillard, René-Jean, 523797 Da Lang Town Dongguan City - Province de Guangdong (CN)
(72) Inventeur: GUILLARD, René-Jean, Dong Guan City Province de Guang Dong (CN)
(74) Mandataire: Degret, Jacques
(86) Numéro de dépôt international: PCT/IB2014/001234
(87) Numéro de publication internationale: WO 2014/207547

(56) Documents cités:
- GB-A- 2 484 530
- US-A1- 2008 050 800
- US-A1- 2009 250 393
- US-A1- 2009 294 354
- CHONGRAK POLPRASERT ET AL: "PRODUCTIVE UTILIZATION OF PIG FARM WASTES: A CASE STUDY FOR DEVELOPING COUNTRIES", RESOURCES CONSERVATION AND RECYCLING, ELSEVIER SCIENCE PUBLISHER, AMSTERDAM, NL, vol. 11, no. 1/04, 30 juin 1994 (1994-06-30), pages 245-259, XP000453896, ISSN: 0921-3449, DOI: 10.1016/0921-3449(94)90093-0
- MALIK ET AL: "Environmental challenge vis a vis opportunity: The case of water hyacinth", ENVIRONMENT INTERNATIONAL, PERGAMON PRESS, US, vol. 33, no. 1, 16 décembre 2006 (2006-12-16), pages 122-138, XP005806089, ISSN: 0160-4120, DOI: 10.1016/J.ENVINT.2006.08.004

## Description

### DOMAINE TECHNIQUE DE L'INVENTION

La présente invention concerne une installation de traitement et de valorisation d'effluents d'élevage, ainsi qu'un procédé de traitement et de valorisation de ces effluents.

La gestion des effluents venant des élevages, en particulier le lisier des élevages de porcs, est un des problèmes majeurs de cette activité pour les années à venir, notamment avec les problèmes écologiques qu'ils posent et les réglementations mises en place.

### ARRIÈRE PLAN TECHNOLOGIQUE DE L'INVENTION

Les effluents d'élevages d'animaux comme le lisier de porc, riches en éléments nutritifs azote, phosphore et potassium « NPK », sont habituellement épandus sur les terres agricoles comme fertilisants suivant des plans d'épandage contrôlés par des réglementations. Pour limiter les pollutions des sols, des bassins versants et des nappes phréatiques, les taux d'épandage autorisés sont de plus en plus réduits.

On peut aussi obtenir avec ces épandages une concentration desdits éléments NPK sur certains bords de mer, lesquels, avec la chaleur, entraînent la prolifération d'algues vertes, qui sont difficilement valorisables à cause de leurs charges en sel de mer et en sable.

Une autre utilisation de ces effluents est le traitement dans des stations de déshydratation dédiées afin d'obtenir des extraits concentrés en éléments NPK, permettant de les livrer à des clients extérieurs. Cependant, ces traitements nécessitent un apport d'énergie et ils produisent différentes matières qui ne sont pas toutes valorisées sur place. Le bilan écologique peut être médiocre, et les coûts ne sont pas optimisés.

Un procédé de traitement des effluents d'animaux connu, présenté notamment par le document GB-A-2484S30, réalise une méthanisation de ces effluents, produisant principalement du méthane, du gaz carbonique et de la chaleur. Les matières issues de cette méthanisation, alimentent une culture de micro-algues, pour obtenir des produits valorisés.

On peut en particulier réaliser, avec un cogénérateur utilisant le méthane, une production d'énergie électrique consommée sur place, ou réinjectée dans un réseau de distribution d'électricité, et de chaleur utilisée pour des chauffages de bâtiments ou d'activités locales.

Par ailleurs, un autre procédé de traitement connu, présenté notamment par le document US-A1-2009/0294354, utilise le liquide sortant de la culture des micro-algues pour alimenter une culture de plantes, afin de consommer les produits nutritifs de ce liquide.

Un problème qui se pose avec ces procédés est que certaines matières résiduelles produites sont économiquement peu valorisables. De plus, ces activités ne comportent pas un équilibre biologique, et en outre elles ne consomment pas toutes les matières principales produites. Il faut alors traiter les résidus qui peuvent poser des problèmes de transformation, de transport ou d'élimination, ce qui augmente le coût global de traitement des effluents et dégrade le bilan écologique.

En particulier, la méthanisation et la culture des plantes d'eau produisent des boues et une grande quantité d'eau chargée, qu'il faut éliminez, en particulier par rejet des eaux ou par épandage. Cependant ces rejets et ces épandages peuvent entraîner des pollutions des sols ou des eaux ; ils sont donc soumis à des réglementations et sont de plus en plus limités.

### DESCRIPTION GÉNÉRALE DE L'INVENTION

La présente invention a notamment pour but d'éviter ces inconvénients de la technique antérieure. Cela étant, son énorme avantage est que la succession des différentes étapes qu'elle préconise permet de valoriser 100% des entrants et ne produit en revanche aucun déchet hors du site.

Elle propose à cet effet une installation de traitement et de valorisation d'effluents d'élevage, comportant une unité de méthanisation apte à produire et à traiter les biogaz obtenus, une unité de cogénération délivrant de l'électricité et de la chaleur à partir de ces biogaz, une unité de culture hydroponique de micro-algues, dans des photo-bioréacteurs alimentés par une partie de la phase liquide du digestat brut produit par la méthanisation, et un système de séparation apte à délivrer une phase liquide de digestat et une phase solide comprenant les boues de digestat, ladite installation étant caractérisée en ce qu'elle comporte en outre une unité de culture de macrophytes alimentée par l'eau ressortant de l'unité de culture des micro-algues et par une autre partie de la phase liquide du digestat brut produit par la méthanisation, et une unité de lombriculture alimentée par la récolte des macrophytes et par les boues constituant la phase solide du digestat brut de la méthanisation.

Un avantage de cette installation est que l'on réalise avec ces différentes unités, regroupées dans l'installation, et avec de la lumière, voire du soleil, une succession de procédés de transformation comprenant des interactions utilisant une grande partie des matières ainsi que de l'énergie générée qui est renouvelable.

Il est à noter que chacun des procédés qui sont successivement mis en oeuvre par l'invention est un procédé biologique naturel qui peut assurer la dépollution du NPK entrant sur le site.

Cette combinaison permet de valoriser au mieux les produits obtenus, qui sont même entièrement valorisés, de limiter les transports, de réduire les coûts de traitement des effluents et d'optimiser le bilan écologique. De plus, elle a l'énorme avantage de ne produire aucun déchet à éliminez, et elle réalise une captation de gaz carbonique.

En particulier, en alimentant la lombriculture par les boues produites par l'unité de méthanisation et par les plantes produites par la culture des macrophytes, on traite ces boues et on absorbe la grande quantité d'eau délivrée par l'installation, ce qui évite des rejets dans la nature. De plus, cette lombriculture fournit des produits hautement valorisés, à forte valeur économique.

D'une manière générale en ajustant le dimensionnement de chaque unité, on reproduit les différents procédés successifs qui s'accomplissent spontanément dans la nature avec le cycle naturel de la vie, pour résoudre le problème de traitement des effluents.

L'installation de traitement et de valorisation selon l'invention peut de plus comporter une ou plusieurs des caractéristiques suivantes, qui peuvent être combinées entre elles.

Avantageusement, les digesteurs de l'unité de méthanisation sont des bassins couverts par des serres des unités de culture de micro-algues, de macrophytes ou de lombriculture.

Avantageusement, l'unité de culture de macrophytes utilise une plante de la famille « Eichhornia crassipes ».

Avantageusement, les macrophytes sont cultivés dans des serres, hors sol dans des bassins chauffés à environ 25°C.

En particulier, les bassins de culture peuvent comporter une profondeur d'environ 30cm et une largeur d'environ 2m, et former des rangées espacées par un chemin de roulement permettant à une machine de rouler dessus.

Avantageusement, les vers de terre de la lombriculture sont du type « californien ».

Avantageusement, l'installation comporte un système qui maintient le compost de lombriculture avec un pourcentage d'humidité du milieu supérieur à 80%.

Avantageusement, l'unité de lombriculture comporte des moyens de récupération du percolat.

En particulier, le compost de lombriculture peut être disposé dans des serres à même le sol sur une bâche étanche, des drains étant installés pour la récupération du percolat.

Avantageusement, l'unité de lombriculture est chauffée à environ 20°C par une circulation d'eau chaude venant des unités de méthanisation et de cogénération.

L'installation peut comporter de plus une exploitation de bois enrichie par le compost de la lombriculture.

Avantageusement, l'exploitation de bois utilise des saules en rotation courte pour les cycles de taille.

L'invention a aussi pour objet un procédé de traitement et de valorisation d'effluents d'élevage comportant les étapes suivantes : une méthanisation des effluents comprenant un traitement des biogaz obtenus, une cogénération délivrant de l'électricité et de la chaleur à partir de ces biogaz, une culture hydroponique de micro-algues dans des photo-bioréacteurs alimentées par une partie de la phase liquide du digestat brut produit par la méthanisation, une séparation délivrant une phase liquide de digestat et une phase solide comprenant les boues de digestat, une culture de macrophyte alimentée par l'eau ressortant de l'unité de culture des micro-algues et par une autre partie de la phase liquide du digestat brut produit par la méthanisation, et une lombriculture alimentée par la récolte des macrophytes et par les boues constituent la phase solide du digestat brut de la méthanisation.

Les spécifications détaillées de l'invention sont données dans la description qui suit en liaison avec les dessins ci-joints. Il est à noter que ces dessins n'ont d'autre but que celui d'illustrer le texte de la description et qu'ils ne constituent donc en aucune sorte une limitation de la portée de la présente invention.

### BRÈVE DESCRIPTION DES DESSINS

Dans les deux planches de dessins annexes :
- la Figure 1 est un schéma d'une installation selon l'invention ; et
- la Figure 2 est un graphique présentant les différentes étapes de cette installation, comprenant en plus une exploitation de bois.

### DESCRIPTION D'UN MODE DE RÉALISATION PRÉFÉRÉ DE L'INVENTION

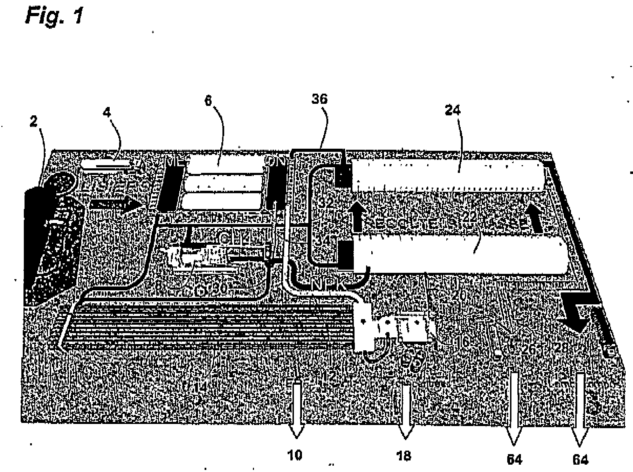

La Figure 1 représente schématiquement une installation de traitement et de valorisation recevant les effluents venant des élevages de la région 2 arrivant par transporteurs 4, ladite installation comportant pour sa partie connue une unité de méthanisation 6 produisant du biogaz 34 délivré à une unité de cogénération 8 produisant de l'électricité utilisée sur l'ensemble du site, l'excès de production étant délivré au réseau extérieur de distribution d'électricité 10.

L'unité de méthanisation, 6 produit des résidus organiques riches en nutriments NPK, dont la phase liquide 12 alimente une unité de culture hydroponique de micro-algues 14 réalisée sous serre du type maraîchère dans des photo-bioréacteurs. La production de cette culture est traitée par un poste de concentration et de conditionnement 16 pour obtenir des produits de valorisation concentrés, facilement transportables, avec une dépense d'énergie réduite, qui sont livrés à des clients extérieurs 18.

Conformément à l'invention, une partie de la phase liquide 12 des résidus organiques ainsi que l'eau 20 provenant de l'unité de culture de micro-algues 14, contenant encore une part de nutriments NPK, sont délivrées à une unité de culture de macrophytes 22 ou plantes aquatiques, réalisée sous serre du type maraîchère.

La production 30 de gaz carbonique CO₂ venant des unités de méthanisation 6 et de cogénération 8 est diffusée vers les unités de culture de micro-algues 14 et de macrophytes 22 pour améliorer les rendements.

La récolte des macrophytes 22 est broyée, puis utilisée pour la lombriculture 24 sous serres se trouvant à côté, qui reçoit aussi des boues de digestat 36 venant de l'unité de méthanisation 6. Les produits issus de la lombriculture 24 donnent un percolat 26, ainsi que du compost de lombriculture 28, qui sont livrés à des clients extérieurs 64.

L'eau chaude 32 produite par les unités de méthanisation 6 et de cogénération 8 est conduite vers les unités de culture 14, 22 et vers la lombriculture 24, pour activer les procédés de transformation.

En complément, l'installation de traitement et de valorisation pourra très avantageusement comporter à proximité des exploitations de bois, notamment des variétés de saules présentant une croissance rapide, qui utilisent le compost produit par la lombriculture 24.

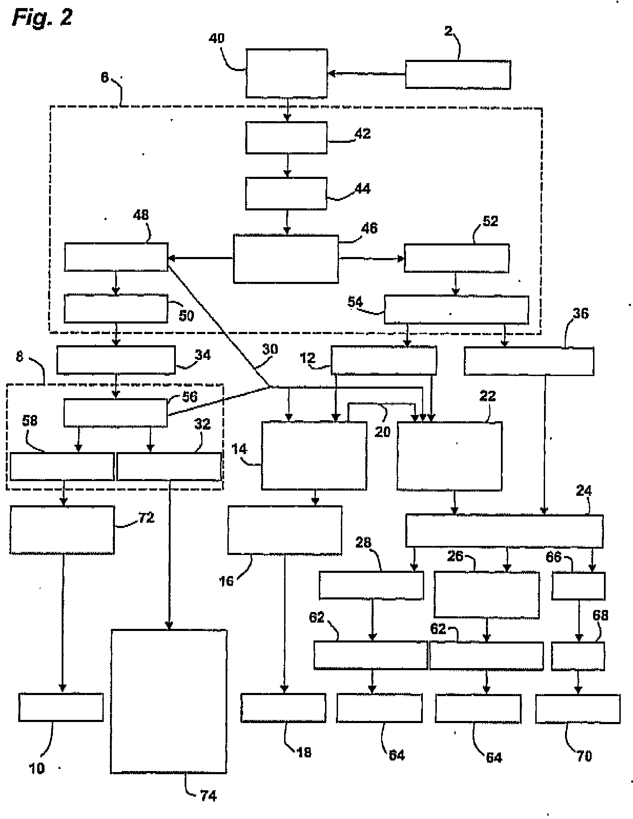

La Figure 2 est un graphique présentant les différents procédés utilisés dans l'installation de traitement et de valorisation qui réalisent une combinaison, chacun de ces procédés utilisant des techniques éprouvées dans des installations en France ou à l'étranger.

La stratégie globale de combinaison des différents procédés utilisés vise à garantir une sécurité optimum des processus de dépollution, en cas de dérèglement d'un des procédés biologiques, et à diversifier la gamme des produits sortants commercialisables, et ce afin d'assurer une meilleure stabilité des recettes de l'entreprise, notamment en cas de variation du cours des produits sur les marchés internationaux.

Pour l'unité de méthanisation, 6, les effluents 40 venant des éleveurs 2 passent par un poste de réception 42 puis par des moyens de stockage 44, avant de réaliser la méthanisation 46.

La méthanisation 46 est une digestion anaérobie qui constitue un procédé naturel en l'absence d'oxygène, et que l'on trouve notamment dans les marais, les rivières, ainsi que dans le système digestif de certains animaux comme les ruminants. La méthanisation, assurée grâce à l'action de bactéries, a pour principal effet de produire du biogaz 34 composé d'environ 60% de méthane CH₄ et 40% de dioxyde de carbone CO₂.

L'unité de méthanisation 6 comporte plusieurs réacteurs appelés digesteurs, délivrant du biogaz brut 48 qui est traité par épuration 50 afin d'éliminer notamment la vapeur d'eau et le sulfure d'hydrogène H₂S, et ce pour donner le biogaz valorisable 34, équivalent à du gaz naturel, mais renouvelable.

Les réacteurs délivrant aussi un résidu organique stabilisé appelé digestat brut 52. Le digestat brut 52, comportant l'intégralité des nutriments NPK présents dans le lisier, mais sous une forme plus assimilable par les plantes, passe par un système de séparation 54 comprenant une décantation, une filtration et une hygiénisation, pour délivrer une phase liquide de digestat 12 et une phase solide comportant les boues de digestat 36.

Le procédé de méthanisation utilisé est du type mésophile, comprenant une température comprise entre 32 à 40°C, qui est moins sensible et plus stable que d'autres procédés comprenant des gammes de température resserrées et plus élevées. On privilégié ainsi la stabilité des procédés au détriment d'une productivité plus importante, afin de garantir une dépollution ininterrompue et un apport constant en énergie thermique pour le bon développement des cultures de l'installation.

De plus, ce procédé mésophile est mieux adapté pour les régions comprenant un climat tempéré, dès lors qu'il évite une consommation d'énergie plus importante pour obtenir des températures plus élevées.

La technologie retenue pour les digesteurs est celle du bassin couvert, qui permet la gestion de gros volumes avec des garanties de sécurité et un coût de construction raisonnable. Cette technique a de plus l'avantage d'assurer une bonne intégration dans les paysages, en évitant des silos en hauteur habituellement utilisés pour la méthanisation par voie humide.

En particulier, on peut réaliser de manière économique des bassins rectangulaires semi-enterrés, creusés dans un sol argileux et rehaussés sur les côtés par la terre excavée, qui reçoivent au fond une membrane étanche.

Avantageusement, on réalise plusieurs bassins connectés les uns aux autres afin d'obtenir une souplesse dans la gestion de ces bassins concernant la maintenance, avec la possibilité de transférer rapidement les effluents d'un bassin vers l'autre sans arrêter le cycle de dépollution, tant en cas de problème que pour les révisions périodique. On obtient alors une meilleure sécurité environnementale.

Avantageusement, on dispose les bassins sous des serres des unités de culture de micro-algues 14, de macrophytes 22 ou de lombriculture 24, de manière à réaliser une isolation thermique supplémentaire de la surface de ces bassins permettant de garantir un bon fonctionnement de la digestion anaérobie, à limiter la surface totale au sol de l'installation complète et à réduire l'impact visuel sur le paysage.

Le biogaz valorisé 34 est délivré à l'unité de cogénération 8, comprenant un cogénérateur 56 disposant d'un moteur qui entraîne un alternateur délivrant une puissance électrique 58 permettant de couvrir les différents besoins en électricité de l'installation de traitement, le surplus 72 étant vendu au réseau de distribution électrique extérieur 10. Pour des raisons de sécurité, on met une certaine distance entre l'unité de cogénération 8 et l'unité de méthanisation 6.

L'unité de méthanisation, 6 et l'unité de cogénération 8 produisent de la chaleur sous forme d'un débit d'eau chaude 32, qui est valorisée toute l'année sur le site 74 par un chauffage des serres de culture des micro-algues 14, des macrophytes 22 et de lombriculture 24. La chaleur est aussi utilisée pour maintenir en température les digesteurs de l'unité de méthanisation. On réalise ainsi des procédés travaillant à des température sensiblement constantes tout au long de l'année, ce qui permet de lisser les phénomènes de saison.

Le dioxyde de carbone 30 produit par l'unité de méthanisation, 6 et l'unité de cogénération 8 est valorisé en étant délivré aux cultures des micro-algues 14 et des macrophytes 22, afin d'accélérer les croissances de la biomasse photosynthétique. Le dioxyde de carbone 30 est capté dans les fumées de combustion du cogénérateur 56 pour donner un gaz contenant environ 13% de ce dioxyde, qui est injecté dans les photo-bioréacteurs.

Les micro-algues sélectionnées pour la culture hydroponique de l'unité de culture des micro-algues 14 se développent dans des tubes transparents appelés photo-bioréacteur « PBR », recevant un liquide contenant les éléments nutritifs NPK venant de la phase liquide du digestat 12, ainsi que le dioxyde de carbone 30 dissous dans ce liquide, afin de synthétiser sa biomasse.

Avantageusement, les tubes transparents, réalisés en polyméthacrylate de méthyle « PMMA », présentent un diamètre d'environ 100mm et sont superposés pour former des batteries de rangées disposées parallèlement. L'eau contenue dans les tubes de culture est maintenue à une température de 25°C par l'effet de serre et par un échangeur eau/eau alimenté par le circuit d'eau chaude 32.

Les dosages des apports d'éléments nutritifs NPK et du dioxyde de carbone, et les réglages de la température, sont pilotés par des systèmes automatisés. On peut en particulier adapter les paramètres pour obtenir des croissances spécifiques riches en certains éléments recherchés suivant les applications, tels les marchés pharmaceutiques, cosmétiques ou alimentaires. Ces paramètres peuvent être le réglage en éléments nutritifs NPK, le dosage en dioxyde de carbone, la température, le réglage de l'acidité pH, le dosage en micro-éléments spécifiques, l'apport en solution carbonée, le réglage de la luminosité et du spectre lumineux, la vitesse de circulation de l'eau et la saturation en gaz de cette eau.

Les variétés d'algues choisies sont douées d'une croissance rapide, qui peut donner un doublement de la masse en une journée, et sont riches en huiles et en éléments valorisables. On obtient des rendements à l'hectare très élevés, qui peuvent être plus de cent fois supérieurs aux rendements des plantes cultivées en terre. La récolte des micro-algues se fait par filtration tangentielle sur une membrane.

Les micro-algues sont traitées dans le poste de concentration et de conditionnement 16 pour être livrées en phase liquide dans des fûts. On obtient ainsi un produit comportant des volumes et des masses limités, ce qui permet de réduire les coûts des transports ainsi que leurs pollutions.

Les micro-algues concentrées sont utilisées en particulier sur les marchés de la cosmétique, avec des extraits lipidiques, protéiniques et des molécules dites anti-âge, de la pharmacie avec les oméga 3/6, de l'alimentation avec des compléments nutritionnels humains ou animales, de l'industrie avec les bio-plastiques et les colorants, et de l'énergie avec des extractions d'huile pour remplacer les énergies fossiles, ou la méthanisation de la biomasse.

De plus, la culture des micro-algues peut générer une production d'oxygène ou d'hydrogène avec certaines souches de micro-algues, ces gaz étant alors récupérés par des échangeur au niveau des tubes photo-bioréacteurs PBR.

La culture des micro-algues absorbe environ 75% des éléments nutritifs NPK, l'eau ressortant des photo-bioréacteurs 20 est délivrée ensuite à la culture hydroponique des macrophytes 22 dans des serres chauffées, pour une étape suivante d'affinage complémentaire de cette eau.

L'unité de culture des macrophytes 22 utilise avantageusement une plante de la famille « Eichhornia crassipes », appelée communément jacinthe d'eau, cultivée hors sol dans des bassins chauffés à environ 25°C. Cette plante aquatique d'origine tropicale implique une culture sous serres dans les régions tempérées ; sa croissance est l'une des plus rapides du règne végétal.

Ce type de plante aquatique a une bonne capacité d'extraction des éléments nutritifs NPK et des métaux lourds contenus dans l'eau, et elle peut réaliser une épuration totale de l'eau polluée, ainsi qu'une diminution de son volume par une transpiration et une évaporation importante.

La culture des macrophytes 22 reçoit à la fois la phase liquide 12 du digestat brut 52, riche en éléments nutritifs NPK, et l'eau 20 venant de la culture des micro-algues 14 contenant encore une part d'éléments nutritifs, pour les épurer. En particulier, 1 hectare de culture de jacinthe d'eau peut épurer environ 250 m² d'eau par jour.

Par ailleurs, cette plante peut être utilisée comme engrais organique, et sa fleur, ses feuilles et ses racines peuvent être utilisées pour l'alimentation animale.

Avantageusement, les bassins de culture disposés dans les serres, comportant une profondeur limitée d'environ 30 cm et une largeur d'environ 2 m, forment des rangées espacées par un chemin de roulement permettant à une machine de rouler dessus pour effectuer de manière automatique les récoltes. Les macrophytes sont récoltés facilement par une machine automatique dédiée, car elles sont flottantes sur l'eau et ne nécessitent pas de coupe ou d'arrachage comme pour les plantes comprenant des racines en terre.

Les macrophytes récoltés sont ensuite broyés dans un hacheur, puis mélangées avec les boues de méthanisation 36, avant d'être épandus sur les cultures de lombrics 24 pour être digérés en fournissant un complément végétal indispensable à la croissance des vers de terre.

La culture sous serres permet de maintenir des conditions de développement optimales toute l'année aux plans de la lumière, de la température et du taux de CO₂. Il est ainsi permis d'assurer une continuité du procédé quelles que soient les saisons et les conditions météorologiques. On peut de plus injecter en atmosphère contrôlée le dioxyde de carbone provenant du biogaz et de la cogénération, pour accélérer la croissance de la plante. Eichhornia crassipes, comme tous les organismes photosynthétiques, a en effet besoin de CO₂ pour assurer sa croissance.

On notera que les macrophytes qui peuvent épurer des eaux très chargées ont un coefficient de production de biomasse parmi les plus élevés pour les plantes aériennes.

La filtration par la culture des lombrics 24 consiste à élever des vers de terre sur un compost formant un support organique arrosé par un liquide chargé de matières organiques, pour réaliser un procédé biologique qui favorise la digestion de ces matières, tout en associant de manière simple une indication biologique de la stabilité du milieu et du bon déroulement du procédé d'épuration.

Le ver de terre respire par contact avec l'eau de son milieu, et il est donc important de garder un pourcentage d'humidité du milieu supérieur à 80%. En particulier, les boues de méthanisation 36 concentrées à 15% de matière sèche permettent de garder une humidité optimum du compost. Avantageusement, on utilise des vers de terre du type « californien », qui sont bien adaptés pour cette fonction.

Naturellement, les lombrics laissent échapper du tube digestif un liquide appelé percolat 26, chargé en bactéries, qui servira d'activateur des sols. Le compost est disposé sous forme de bandes dans des serres du type maraîchère, à même le sol sur une bâche étanche garantissant la récupération du percolat 26 par des drains.

La température optimum de la culture des lombrics 24, qui est de 20°C, est assurée par une circulation d'eau chaude sous la couche de compost.

La croissance de la population des lombrics est assurée par l'apport en végétaux venant de la culture des macrophytes 22, et en boues de méthanisation 36. Une fois le compost arrivé à maturité, les vers de terre sont retirés pour ensemencer des lignes voisines.

Le compost obtenu 28 est retiré puis conditionné 62, le percolat 26 est aussi conditionné 62, ces produits étant valorisés avec une valeur économique importante auprès d'utilisateurs réalisant des cultures biologiques 64.

Les lombrics consomment les phases solides des boues de méthanisation 36. Le percolat chargé à 100% d'humidité ainsi que le compost produit, chargé à environ 85% d'humidité, absorbent une partie des eaux produites par l'installation, en complément des eaux dissipées par évaporation et transpiration par la culture des macrophytes.

En dimensionnant les différents éléments de l'installation, on peut réaliser ainsi une consommation complète des boues ainsi que des eaux produites par cette installation, ce qui évite de les mettre en rejet, en épandage ou en décharge suivant des plans soumis à des normes et à des autorisations pour éviter les pollutions.

On notera qu'un excès d'apport en éléments nutritifs NPK sur une terre peut détruire la faune bactérienne indispensable pour réaliser une absorption de ces éléments par les plantes et peut rendre la terre stérile. La croissance des végétaux est alors impossible. L'utilisation du percolat 26 ou du compost 28 des lombrics permet d'apporter aux sols les bactéries issues du tube digestif du ver de terre, ce sont des transmetteurs ioniques qui redonnent aux plantes la capacité d'absorber les éléments nutritifs NPK présents dans la terre.

La dernière unité de l'installation de traitement et de valorisation conforme à l'invention, mise en complément de manière optionnelle, comportera avantageusement une culture intensive de saules en rotation courte 66, appelée aussi taillis à très courte rotation « TTCR », qui reçoit le compost de la culture des lombrics 24. Son rôle principal est la captation du dioxyde de carbone CO₂, mais cette culture joue aussi un rôle dans l'évaporation naturelle des rejets liquides déjà filtrés par les unités de traitement précédentes, afin de limiter le retour au milieu naturel des eaux par le réseau hydraulique de surface.

Pour une installation traitant entre 110.000 et 146.000 tonnes de lisier de porc par an, soit un traitement moyen de 350 tonnes par jour, avec une capacité installée de 400 tonnes par jour, les estimations donnent une production de micro-algues utilisées par les industries agroalimentaires, pharmaceutiques et cosmétiques, de 2.500 tonnes de matières sèches par an, une production de compost de lombric de 21.000 tonnes par an, et un accélérateur de croissance biologique prêt à l'emploi sous forme liquide fourni par le percolat de lombric de 9.000 tonnes par an. La surface de culture des jacinthes d'eau prévue est de 5 ha.

Cette installation permet d'absorber par évaporation naturelle, ou par exportation à travers les produits valorisés, l'intégralité des 350 tonnes par jour de liquide entrant dans le procédé.

Elle réalise en particulier une valorisation de terres agricoles de faible valeur agronomique, comme les landes ou les terres peu fertiles, grâce au système de culture hors sol.

Comme il va de soi, l'invention ne se limite pas aux seuls modes d'exécution préférentiels décrits ci-dessus.

Elle embrasse au contraire toutes les variantes possibles de réalisation, pour autant que ces dernières ne sortent pas du cadre délimité par les revendications ci-jointes qui définissent la portée de la présente invention.

## Revendications

1. Installation de traitement et de valorisation d'effluents d'élevage (2), comportant une unité de méthanisation des effluents (6) apte à produire et à traiter les biogaz obtenus (48), une unité de cogénération (8) délivrant de l'électricité (58) et de la chaleur (32) à partir de ces biogaz, une unité de culture hydroponique de micro-algues (14), dans des photo-bioréacteurs alimentés par une partie de la phase liquide (12) du digestat brut (52) produit par la méthanisation, et un système de séparation (54) apte à délivrer une phase liquide (12) de digestate et une phase solide (36) comprenant les boues de digestat, **caractérisée en ce qu'**elle comporte en outre une unité de culture de macrophytes (22), alimentée par l'eau (20) ressortant de l'unité (14) de culture des micro-algues et par une autre partie de la phase liquide (12) du digestat brut (52) produit par la méthanisation, et une unité de lombriculture (24) alimentée par la récolte des macrophytes (22) et par les boues (36) constituant la phase solide du digestat brut (52) de la méthanisation (46).

2. Installation selon la revendication 1, **caractérisée en ce que** les digesteurs de l'unité de méthanisation (6) sont des bassins couverts par des serres des unités de culture de micro-algues (14), de macrophytes (22) ou de lombriculture (24).

3. Installation selon la revendication 1 ou 2, **caractérisée en ce que** l'unité de culture de macrophytes (22) utilise une plante de la famille « Eichhornia crassipes ».

4. Installation selon l'une quelconque des revendications précédentes, **caractérisée en ce que** les macrophytes sont cultivés dans des serres, hors sol dans des bassins chauffés à environ 25°C.

5. Installation selon la revendication 4, **caractérisée en ce que** les bassins de culture comportent une profondeur d'environ 30cm et une largeur d'environ 2m, et forment des rangées espacées par un chemin de roulement permettant à une machine de rouler dessus.

6. Installation selon l'une quelconque des revendications précédentes, **caractérisée en ce que** les vers de terre de la lombriculture (24) sont du type « californien ».

7. Installation selon l'une quelconque des revendications précédentes, **caractérisée en ce qu'**elle comporte un système qui maintient le compost de lombriculture (24) avec un pourcentage d'humidité du milieu supérieur à 80%.

8. Installation selon l'une quelconque des revendications précédentes, **caractérisée en ce que** l'unité de lombriculture (24) comporte des moyens de récupération du percolat (26).

9. Installation selon la revendication 8, **caractérisée en ce que** le compost de lombriculture (24) est disposé dans des serres à même le sol sur une bâche étanche, des drains étant installés pour la récupération du percolat (26).

10. Installation selon l'une quelconque des revendications précédentes, **caractérisée en ce que** l'unité de lombriculture (24) est chauffée à environ 20°C par une circulation d'eau chaude venant des unités de méthanisation (6) et de cogénération (8).

11. Installation selon l'une quelconque des revendications précédentes, **caractérisée en ce qu'**elle comporte une exploitation de bois (66) enrichie par le compost de la lombriculture (24).

12. Installation selon la revendication 11, **caractérisée en ce que** l'exploitation de bois utilise des saules en rotation courte (66) pour les cycles de taille.

13. Installation selon l'une quelconque des revendications 1 à 12, **caractérisée en ce qu'**elle comprend des moyens permettant de récupérer le gaz carbonique CO₂ contenu dans les biogaz et de diffuser ce gaz vers l'unité (14) de culture de micro-algues et vers l'unité (22) de culture de macrophytes.

14. Procédé de traitement et de valorisation d'effluents d'élevage (2), **caractérisé en ce qu'**il comporte les étapes suivantes : une méthanisation (46) des effluents comprenant un traitement des biogaz obtenus (48), une cogénération (56) délivrant de l'électricité (58) et de la chaleur (32) à partir de ces biogaz, une culture hydroponique de micro-algues (14) dans des photo-bioréacteurs alimentés par une partie de la phase liquide (12) du digestat brut (52) produit par la méthanisation, une séparation délivrant une phase liquide (12) de digestat et une phase solide (36) comprenant les boues de digestat, une culture de macrophytes (22) alimentée par l'eau (20) ressortant de l'unité (14) de culture des micro-algues et par une autre partie de la phase liquide (12) du digestat brut (52) produit par la méthanisation, et une lombriculture (24) alimentée par la récolte des macrophytes (22), et par les boues (36) constituant la phase solide du digestat brut (52) de la méthanisation (46).

## Patentansprüche

1. Anlage zur Behandlung und Wiederverwertung von Viehzuchtexkrementen (2), mit einer Methanisierungseinheit (6) der Exkremente, die in der Lage ist, die Biogase (48) zu erzeugen und die erhaltenen zu verarbeiten, einer Co-Erzeugungseinheit (8), die Elektrizität (58) und Wärme (32) ausgehend von diesen Biogasen liefert, einer hydroponischen Kultivierungseinheit (14) von Mikroalgen in Foto-Bioreaktoren, die von einem Teil der Flüssigphase (12) des rohen Gärguts (52), erzeugt durch die Methanisierung, gespeist wird, und mit einem Trennungssystem (54), das in der Lage ist, eine Flüssigphase (12) des Gärguts und eine feste Phase (36) zu liefern, die die Gärgutschlämme aufweist, **dadurch gekennzeichnet, dass** sie darüber hinaus eine Kultivierungseinheit von Makrophyten (22) aufweist, die von Wasser (20) gespeist werden, das aus der Kultivierungseinheit (14) der Mikroalgen und durch einen anderen Teil der Flüssigphase (12) des rohen Gärguts (52) gespeist wird, erzeugt durch die Methanisierung, und eine Wurmkultureinheit (24) aufweist, die von der Ernte der Makrophyten (22) und durch den Gärschlamm (36) gespeist wird, der die feste Phase des rohen Gärguts (52) der Methanisierung (46) bildet.

2. Anlage nach Anspruch 1, **dadurch gekennzeichnet, dass** die Vergärungsanlagen der Methanisierungseinheit (6) Becken sind, die mit Gewächshäusern der Kultivierungseinheiten (14) der Mikroalgen, der Makrophyten (22) oder der Wurmkultur (24) bedeckt sind.

3. Anlage nach Anspruch 1 oder 2, **dadurch gekennzeichnet, dass** die Einheit der Makrophytenkultivierung (22) eine Pflanze der Familie "Eichhornia crassipes" verwendet.

4. Anlage nach einem der vorhergehenden Ansprüche, **dadurch gekennzeichnet, dass** die Makrophyten in Gewächshäusern außerhalb des Bodens in auf 25°C aufgeheizten Becken kultiviert werden.

5. Anlage nach Anspruch 4, **dadurch gekennzeichnet, dass** die Kultivierungsbecken eine Tiefe von ungefähr 30 cm und eine Breite von ungefähr 2 m aufweisen und durch einen Fahrweg beabstandete Reihen bilden, der einer Maschine ermöglicht, darüber zu fahren.

6. Anlage nach einem der vorhergehenden Ansprüche, **dadurch gekennzeichnet, dass** die Regenwürmer der Wurmkultur (24) vom Typ "kalifornisch" sind.

7. Anlage nach einem der vorhergehenden Ansprüche, **dadurch gekennzeichnet, dass** sie ein System aufweist, das den Kompost der Wurmkultur (24) mit einem Milieufeuchtegehalt von über 80% beibehält.

8. Anlage nach einem der vorhergehenden Ansprüche, **dadurch gekennzeichnet, dass** die Wurmkultureinheit (24) Einrichtungen zur Gewinnung von Perkolat (26) aufweist.

9. Anlage nach Anspruch 8, **dadurch gekennzeichnet, dass** der Kompost der Wurmkultur (24) in Gewächshäusern auf der Höhe des Bodens auf einer dichten Abdeckplane verteilt ist, wobei für die Wiedergewinnung des Perkolats (26) Durchlässigkeitsbereiche angeordnet sind.

10. Anlage nach einem der vorhergehenden Ansprüche, **dadurch gekennzeichnet, dass** die Wurmkultureinheit (24) auf ungefähr 20°C erwärmt wird durch eine Zirkulation von warmem Wasser, das von Methanisierungseinheiten (6) und der Co-Erzeugungseinheit (8) herrührt.

11. Anlage nach einem der vorhergehenden Ansprüche, **dadurch gekennzeichnet, dass** sie eine Holzverarbeitung (66) aufweist, die durch den Kompost der Wurmkultur (24) angereichert ist.

12. Anlage nach Anspruch 11, **dadurch gekennzeichnet, dass** die Holzverarbeitung Weiden in kurzer Drehung (66) für die Schnittzyklen verwendet.

13. Anlage nach einem der Ansprüche 1 bis 12, **dadurch gekennzeichnet, dass** sie Einrichtungen aufweist, die ermöglichen, das Kohlenstoffgas CO₂ wiederzugewinnen, das in den Biogasen enthalten ist, und dieses Gas zur Einheit (14) der Kultivierung der Mikroalgen und zur Einheit (22) der Kultivierung der Makrophyten zu verteilen.

14. Verfahren zur Behandlung und Wiederverwertung von Viehzuchtexkrementen (2), **dadurch gekennzeichnet, dass** es die folgenden Schritte aufweist: Eine Methanisierung (46) der Exkremente mit einer Verarbeitung der erhaltenen Biogase (48), einer Co-Erzeugung (56), die Elektrizität (58) und Wärme (32) liefert ausgehend von diesen Biogasen, einer hydroponischen Mikroalgenkultivierung (14) in Foto-Bioreaktoren, die von einem Teil der Flüssigphase (12) des rohen Gärguts (52) gespeist wird, das durch die Methanisierung erzeugt wird, einer Trennung, die eine Flüssigphase (12) des Gärguts und eine feste Phase (36) liefert, die die Schlämme des Gärguts enthält, eine Makrophytenkultivierung (22), die von dem Wasser (20) gespeist wird, das aus der Einheit (14) der Mikroalgenkultivierung und von einem anderen Teil der Flüssigphase (12) des rohen Gärguts (52) stammt, erzeugt durch die Methanisierung, und eine Wurmkultivierung (24), die durch die Ernte der Makrophyten (22) und durch deren Schlämme (36) gespeist wird, die die feste Phase des rohen Gärguts (52) der Methanisierung (46) bildet.

## Claims

1. Facility for treating and recycling animal waste (2), comprising an effluent methanization unit (6) capable of producing and treating the biogas obtained (48), a cogeneration unit (8) providing electricity (58) and heat (32) from these biogas, a hydroponic unit for cultivating microalgae (14) in photo-bioreactors fed by part of the liquid phase (12) of the raw digestate (52) produced by the methanization, and a system of separation (54) capable of supplying a digestate liquid phase (12) and a solid phase (36) comprising digestate sludge, **characterized in that** it further comprises a macrophyte culture unit (22) fed by the water (20) emerging from the microalgae culture unit (14) and by another part of the liquid phase (12) of the raw digestate (52) produced by the methanization, and a vermiculture unit (24) fed by the harvested macrophytes (22) and by the sludge (36) constituting the solid phase of the raw digestate (52) from methanization (46).

2. Facility according to claim 1, **characterized in that** the digesters of the methanization unit (6) are pools covered by greenhouses of the microalgae (14), macrophyte (22) or vermiculture (24) culture units.

3. Facility according to claim 1 or 2, **characterized in that** the macrophyte culture unit (22) uses a plant of the family *Eichhornia crassipes.*

4. Facility according to any one of the preceding claims, **characterized in that** the macrophytes are grown in greenhouses, above ground in pools heated to about 25°C.

5. Facility according to claim 4, **characterized in that** the culture pools have a depth of about 30cm and a width of about 2m, and form rows separated by a runway for a machine to roll on.

6. Facility according to any one of the preceding claims, **characterized in that** the earthworms of the vermiculture (24) are of the "Californian" type.

7. Facility according to any one of the preceding claims, **characterized in that** it comprises a system that keeps the compost from the vermiculture (24) at a humidity percentage of the medium that is greater than 80%.

8. Facility according to any one of the preceding claims, **characterized in that** the vermiculture unit (24) includes means for recovering the percolate (26).

9. Facility according to claim 8, **characterized in that** the compost from the vermiculture (24) is placed in greenhouses directly on the ground on a waterproof tarpaulin, with drains being installed for recovering the percolate (26).

10. Facility according to any one of the preceding claims, **characterized in that** the vermiculture unit (24) is heated to about 20°C by a hot water circulation coming from the methanization (6) and cogeneration (8) units.

11. Facility according to any one of the preceding claims, **characterized in that** it comprises a timber operation (66) enriched by the compost from the vermiculture (24).

12. Facility according to claim 11, **characterized in that** the timber operation uses willow in short rotation (66) for pruning cycles.

13. Facility according to any one of the claims 1 to 12, **characterized in that** it comprises means allowing the carbon dioxide gas CO2 contained in the biogas to be recovered and this gas to flow to the microalgae culture unit (14) and to the macrophyte culture unit (22).

14. Method for treating and recycling animal waste (2), **characterized in that** it comprises the following steps: a methanization (46) of the effluents comprising a processing of the biogas obtained (48), a cogeneration (56) supplying electricity (58) and heat (32) from these biogas, a hydroponic microalgae culture (14) in photo-bioreactors fed by part of the liquid phase (12) of the raw digestate (52) produced by the methanization, a separation supplying a digestate liquid phase (12) and a solid phase (36) comprising digestate sludge, a macrophyte culture (22) fed by the water (20) emerging from the microalgae culture unit (14) and by another part of the liquid phase of the raw digestate (52) produced by the methanization, and a vermiculture (24) fed by the harvested macrophytes (22), and by the sludge (36) constituting the solid phase of the raw digestate (52) from methanization (46).
